Europäisches Patentamt

European Patent Office

Office européen des brevets

(11) Publication number: **0 101 617**
**B1**

(12) **EUROPEAN PATENT SPECIFICATION**

(45) Date of publication of patent specification: **18.04.90**

(21) Application number: **83108220.1**

(22) Date of filing: **19.08.83**

(51) Int. Cl.⁵: **C 12 N 15/00, C 12 P 21/02,**
**C 12 N 5/00 // C12R1/91**

(54) Recombinant DNA containing a hepatitis B virus gene, mammalian cells transformed with the cloned viral DNA, and production of hepatitis B virus proteins.

(30) Priority: **20.08.82 JP 145092/82**

(43) Date of publication of application:
**29.02.84 Bulletin 84/09**

(45) Publication of the grant of the patent:
**18.04.90 Bulletin 90/16**

(84) Designated Contracting States:
**AT BE CH DE FR GB IT LI NL SE**

(56) References cited:
EP-A-0 022 685
EP-A-0 038 765
EP-A-0 073 656

Journal of Medical Virology (1981) 8, p. 237-43.
EUKARYOTIC VIRAL VECTORS, CONFERENCE,
Cold Spring Harbor, N.Y., US, December 3-6,
1981, pages 55-60, edited by Y. Gluzman in
1982; C.-C. LIU et al.: "Expression of hepatitis B
surface antigen using lytic and nonlytic SV40-
based vectors"

(73) Proprietor: **Juridical Foundation The Chemo-**
**Sero-Therapeutic Research Institute**
**668, Okubo Shimizu-machi**
**Kumamoto-shi Kumamoto-ken (JP)**

(72) Inventor: **Nozaki, Chikateru**
**622-8 Shinchi Shimizu-machi**
**Kumamoto-shi Kumamoto-ken (JP)**
Inventor: **Miyanohara, Atsushi**
**8-16 Kori-motomachi**
**Neyagawa-shi Osaka-fu (JP)**
Inventor: **Hamada, Fukusaburo**
**2679-2 Suya, Nishigoshi-machi**
**Kikuchi-gun Kumamoto-ken (JP)**
Inventor: **Ohtomo, Nobuya**
**6-22-29 Shimasaki**
**Kumamoto-shi Kumamoto-ken (JP)**
Inventor: **Matsubara, Kenichi**
**3-1-57-909 Fukushima Fukushima-ku**
**Osaka-shi Osaka-fu (JP)**

(74) Representative: **Vossius & Partner**
**Siebertstrasse 4 P.O. Box 86 07 67**
**D-8000 München 86 (DE)**

Courier Press, Leamington Spa, England.

# EP 0 101 617 B1

(56) References cited:

CHEMICAL ABSTRACTS, vol. 93, no. 21, 1980,
page 358, no. 200838m, Columbus, Ohio, US N.
HSIUNG et al.: "Cotransfer of circular and linear
prokaryotic and eukaryotic DNA sequences into
mouse cells" & PROC. NATL. ACAD. SCI. U.S.A.
1980, 77(8),4852-6

M. Fried & E. Ruley - Eukaryotic - Vectors ed. by
Y Cluzman (1982) cold spring - Harbor
Laboratory - pages 67-70

## Description

The present invention relates to recombinant DNA molecules which permit the simultaneous production of the Hepatitis B virus surface antigen (HBsAg) and the Hepatitis B virus e antigen (HBeAg), to mammalian cells transformed with them and to a method for simultaneous production of HBsAg and HBeAg.

Hepatitis B is usually caused by transfusing blood of Hepatitis B virus (HBV) positive patients and there is no drug suitable for complete remedy.

The most suitable prophylaxis is provided by vaccines containing the HBsAg. However, it is very difficult to produce the HBsAg vaccine in a technical scale, because HBV is infectious only to humans and chimpanzees (one has never succeeded to infect a cell culture with HBV). Due to this specificity of HBV, HBsAg can be obtained only from human donors.

It is known to prepare only HBsAg by recombinant DNA technique in *E. coli* as the host organism; see Japanese Patent Laid Open Application No. 104887/1980. However, according to this method using *E. coli,* it is still difficult to produce the desired HBsAg in a large scale, because the produced HBsAg is easily degraded within *E. coli* and the further growth of *E. coli* is inhibited by the HBsAg produced. This results in a low productivity of HBsAg.

It is also known to transform certain animal cells with a cloned HBV DNA; see Japanese Patent Laid Open Application No. 39784/1982. The recombinant DNA used in this method is prepared by inserting HBV DNA into the *E. coli* plasmid pBR322. However, as the site inhibiting the replication in mammalian cells is not deleted and no DNA which can replicate in mammalian cells (e.g. SV40 DNA) is inserted into said plasmid pBR322, the obtained recombinant DNA when introduced into mammalian cells, for instance monkey cells (e.g. COS Cells; see Gluzman, Y.; Cell, *23*, 175—182, 1981), does not effectively replicate in these cells.

Liu and Levinson, "Eukaryotic Viral Vectors", Y. Gluzman ed., Cold Spring Harbor Laboratory (1982), p. 55 to 60 describe the expression of HBsAg in mammalian host cells. The DNA fragment which is contained in the expression vector is merely a part of the HBV genome which encodes HBsAg but not HBeAg. Furthermore, the disclosed expression system in which a lytic vector is used destroys the host cells during the production of HBsAg. Thus, it is not possible to subculture the transformed mammalian host cells and to continuously produce HBsAg. The disclosed expression system in which non-lytic vectors are used does not allow the continuous production of HBsAg either, because these vectors are lost when the transformed mammalian host cells are subcultured.

Fried et al., "Eukaryotic Viral Vectors", Y. Gluzman ed., Cold Spring Harbor Laboratory (1982), p. 67 to 70, only described that recombinant plasmids containing a HSV TK gene, a polyoma virus replication initiation site and a promoter can be used for the transformation of animal cells.

Mackay et al., J. Med. Virol. 8 (1981), 237 to 243 describe the production of the HBeAg by proteolytic degradation of the Hepatitis B core antigen (HBcAg) expressed in E. coli. The DNA fragment which is contained in the expression vector is merely a part of the HBV genome which encodes HBcAg but not HBsAg.

Du Bois et al., Proc. Natl. Acad. Sci. USA 77 (1980), p. 4549—4553, describe the expression of HBsAg in mouse cells. The expression vector contains HbsAg and HBcAg encoding genes which are arranged as follows:

HBs gene—HBc gene—HBs gene—HBc gene.

Due to this gene arrangement the transformed mouse cells only produce HBsAg but not HBcAg or HBeAg.

Accordingly, the technical problem underlying the present invention is to provide recombinant DNA molecules which permit the simultaneous production of HBsAg and HBeAg (after the primary expression of HBcAg) in transformed mammalian host cells and which can stably repliate in these cells as well as in E. coli.

The HBV proteins produced by the transformed mammalian host cells of the present invention have the same immunological properties as natural HBV proteins obtained from human blood serum and they can be produced in a large scale.

An object of the present invention is to provide novel recombinant DNA molecules as specied in the claims which contain HBsAg and HBcAg genes in a particular arrangement and which are suitable for the stable transformation of mammalian cells. Another object of the invention is to provide mammalian cells transformed with the recombinant DNA, particularly transformed mouse L cells. A further object of the invention is to provide a method for simultaneously producing HBV proteins in a technical scale using said transformed mammalian cells. These and other objects and advantages of the invention will become apparent to persons skilled in the art from the following description.

The novel recombinant DNA of the present invention can be prepared by inserting HBV genes into a shuttle vector, e.g. pXRIIG (cf. Lusky, M., Nature *293*, 79, 1981). Said novel recombinant DNA can be used for transforming mammalian cells, preferably thymidine kinase deficient (TK⁻) mammalian cells (e.g. mouse LTK⁻ cells). The present invention is characterized in that the HBs antigen and the HBeAg are produced simultaneously by the transformed mammalian host cells.

The production of the recombinant DNA, of the transformed mammalian cells, and of the HBV proteins is illustrated below in more detail.

(1) HBV genome

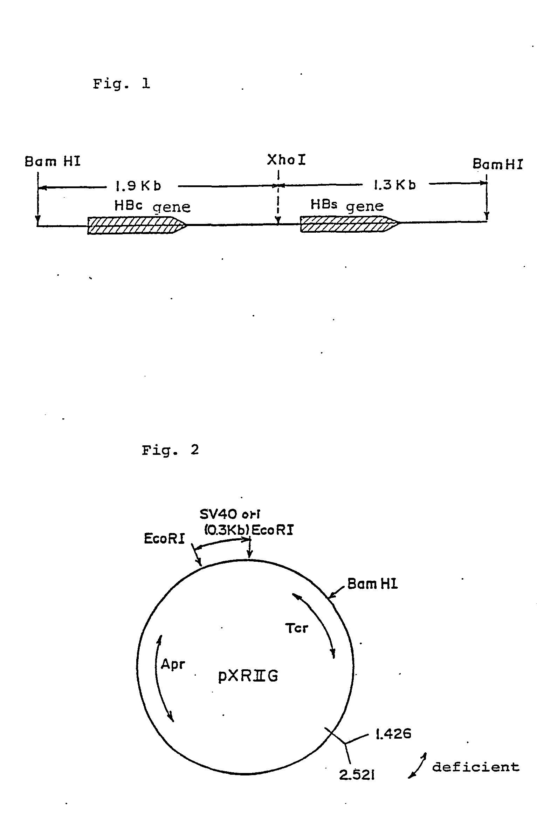

The HBV genome is preferably the genomic DNA of the HBV subtype adr which is frequently observed in Japan and also in countries of Southeast Asia and is cloned in *E. coli*. This HBV DNA contains a unique recognition site for the restriction enzymes XhoI and BamHI. A fragment having terminal BamHI sites, which is obtained by clearing the HBV genome with BamHI has the structure as shown in Figure 1, wherein the HBc gene and the HBs gene are present in the same orientation.

A fragment having terminal XhoI sites is obtained by cleaving the HBV genome with XhoI. This fragment contains the HBs and the HBc gene. Furthermore, a fragment containing only the HBs gene may be obtained by cleaving the XhoI site-terminating fragment with BamHI.

The HBV DNA is prepared as follows:

Viral particles (Dane particles) are isolated from blood of a person who is positive for HBsAg. The HBV DNA (3,200 bp) usually is a partly double-stranded circular DNA. Between 15 to 50% of the HBV DNA is single-stranded. Accordingly, the HBV DNA is treated with an endogenous DNA polymerase by the method of Sattler and Robinson (cf. F. Sattler & W. W. Robinson, Journal of Virology, *32*, 226—233, 1979, "Hepatitis B viral DNA molecules have cohesive ends") in order to change the single-stranded region to a double-stranded DNA suitable for cloning the genome. After filling in the partially single stranded DNA the double-stranded DNA is extracted, and propagated by molecularly cloning in *E. coli*. This molecularly cloned double-stranded HBV DNA is treated with an appropriate restriction enzyme to give a fragment which is used for the construction of the desired plasmid.

The HBV DNA used is preferably of subtype adr which is frequently observed in Japan and in other countries in Southeast Asia, but may be also HBV DNA of the subtype adw or ayw which is frequently observed in European countries and the U.S.A.

(2) Vector

The vector used in the present invention is an *E. coli* plasmid containing the replication origin of SV40 DNA, which can replicate both in *E. coli* and in mammalian cells. The *E. coli* plasmid includes any conventional plasmids, for example, originating from ColE1, pMB1, (cf Hershfield, V., Proc. Natl. Acad. Sci, U.S.A., *71*, 3455, 1974) and plasmids originating from p15A (cf. Chang, A.C.Y., J. Bact., *134*, 1141, 1978).

The SV40 DNA to be inserted into the *E. coli* plasmid is a DNA of a virus which is well known as a mammalian cancer virus being capable of infecting and inducing cancer in monkeys. For use in recombinant DNA techniques the SV40 DNA is usually recombined with another DNA, for instance with DNA coding for β-globin. The recombinant DNA thus obtained is introduced into cultured monkey cells in order to produce β-globin (cf. Mulligan, R. C., Nature *277*, 108, 1979). In the present invention, the origin of replication of the SV40 DNA (it is usually referred to as "SV40 ori") is inserted into an *E. coli* plasmid which is then used for preparation of a recombinant DNA containing HBV genes. The recombinant DNA thus prepared can replicate in both *E. coli* and COS cells.

The vector used in the present invention is preferably deficient in the replication-inhibiting region. A preferred example of such a vector DNA is a recombinant DNA consisting of *E. coli* plasmid pBR 322 deficient in the region toxic to mammalian cells (i.e. region of inhibiting replication within COS cells; 1.426—2.521 kb) (said plasmid pBR322 being designed "pXf³") and of the replication origin (0.31 kb) of SV40 DNA. This recombinant plasmid is designated "pXRIIG" and has the structure shown in Figure 2. The origin of replication (0.31 kb) of SV40 DNA is inserted into the EcoRI cleavage site of pBR322, and the recombinant DNA is deficient in the region (1.426—2.521 kb) inhibiting the replication in mammalian cells and optionally deficient also in another unnecessary regions (e.g. 3.102—3.211 kb) in order to reduce the size of the vector. The recombinant DNA contains a tetracycline-resistance gene (Tcʳ) and an ampicillin-resisitance gene (Apʳ). These antibiotic-resistance genes are useful as selective markers in the preparation of transformed *E. coli*. Various other antibiotic-resistance genes, e.g. a kanamycin-resistance gene or a chloramphenicol-resistance gene, may also be useful.

The vector is used for the preparation of a recombinant DNA containing HBV DNA in the form of a fragment after being cleaved with an appropriate restriction enzyme, such as BamHI, SalI, HindIII, etc.

(3) Constructoin of recombinant DNA (HBV gene-expression plasmid)

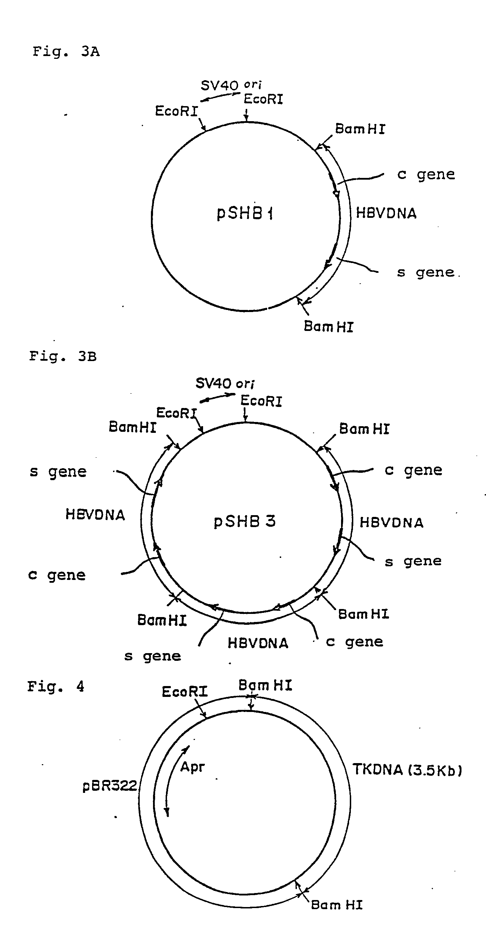

The fragment obtained by cleaving the above vector with an appropriate restriction enzyme (e.g. BamHI) is recombined with a fragment containing the HBV genome in an appropriate ratio to give the desired recombinant DNA. By varying the ratio of each fragment, various recombinant DNAs can be obtained wherein one fragment of the vector is combined with one or more of the fragments of the HBV DNA. Usually, the vector fragment and the fragment of the HBV DNA are used in a molar ratio of 1:1 to 1:10. Preferred recombinant DNAs consist of 2 to 4 fragments of HBV DNA and one vector fragment. The recombinant DNAs have a structure as shown in Figure 3, wherein Figure 3A shows a recombinant DNA consisting of one BamHI fragment of the HBV DNA and one fragment of the vector (designated "pSHB1") and Figure 3B shows a recombinant DNA consisting of three BamHI fragments of HBV DNA and one fragment of the vector (designated "pSHB3"). As is shown in Figure 3, the HBV DNA fragments are inserted in the same orientation, i.e. in the head-to-tail tandem form.

(4) Transformation of mammalian cells

The above recombinant DNA containing the HBV DNA is introduced into mammalian cells together with DNA coding for TK. Then the transformed mammalian cells are cultivated.

The mammalian cells are preferably TK⁻ cells. e.g. mouse LTK⁻ cells, because in this way the desired transformed cells can selectively be isolated after the transformation is carried out by treating the cells with the recombinant DNA together with several ten to several hundred times of the DNA coding for TK. There may also be used a recombinant DNA containing the DNA coding for TK instead of using the recombinant DNA and the DNA coding for TK separately.

The transformation is more specifically illustrated below using mouse LTK⁻ cells as an example of mammalian cells.

Mouse LTK⁻ cells are cultivated in Dulbecco's modified Eagle medium (hereinafter, referred to as "DMEM") supplemented with 10% calf serum (cf. Dulbecco, R. & Freeman, G.; Virology, *8*, 396, 1959) and thereto is added a solution of the recombinant DNA and the DNA coding for TK in an aqueous calcium phosphate solution. The mixture is allowed to stand at room temperature for a few or several ten of minutes, usually for about 30 minutes. Additional DMEM is added to the resulting mixture, and the mixture is cultivated for 4 to 5 hours. After substituting by new DMEM, the mixture is further cultivated for 12 to 24 hours. The resulting culture is further cultivated in a medium containing hypoxanthine (15 µg/ml), aminopterin (1 µg/ml) and thymidine (5 µg/ml) (hereinafter, referred to as "HAT medium") [cf. Littlefield; J. Proc. Natl. Acad. Sci. USA, *72*, 3961—3965, 1963] to give the desired transformed mouse L cells. In the above transformation procedure, the starting mouse LTK⁻ cells are deficient in the DNA coding for TK and hence can not grow in HAT medium, but on the other hand, the transformed mouse L cells containing the DNA coding for TK are able to synthesize thymidine kinase and hence can grow in HAT medium. Accordingly, only the desired transformed cells are selectively isolated by cultivating in the above HAT medium.

(5) DNA coding for TK

DNA coding for TK is used in the above transformation of mammalian cells together with a recombinant DNA containing the HBV DNA. The DNA coding for TK is a recombinant DNA of an E. coli plasmid (e.g. pBR322) and a DNA coding for TK derived from Herpes simplex virus and has the structure as shown in Figure 4 [cf. Florence Colbere-Garapin, 3rd General Meeting of ESACT, Oxford 1979, Develop. biol. Standard, *46*, 75—82, 1980].

(6) Culture of transformed mammalian cells and production of HBV protein

The transformed mammalian cells obtained above are cultivated in an appropriate medium in a usual manner to simultaneously produce large amounts of HBsAg and HBeAg within the cultivated cells. The proteins are released into the medium. In this culture, a mixture of "s" antigen and "e" antigen is produced, but they can easily be separated by conventional protein purification methods, for instance, by passing the culture broth through a column containing an anti-HBs antibody and then eluting the "s" antigen with a 0.1 M HCl-glycine buffer, and likewise by passing the culture broth through a column containing an anti-HBe antibody and then eluting the "e" antigen therefrom with the same buffer as above.

The HBV proteins thus obtained have the same immunological properties as the natural HBV proteins originated from human blood serum and can be used for the preparation of HBV vaccines and diagnostic reagents like the natural HBV proteins.

The recombinant DNA, the transformed mammalian cells and the method for the simultaneous production of HBV proteins of the present invention are illustrated by the following examples.

Example 1
(1) Preparation of HBV DNA
(i) Preparation of virus DNA

Pooled blood plasma (700 ml) obtained from ten patients who are positive in HBsAg (subtype adr) and HBcAg is centrifuged at 5,000 r.p.m. for 20 minutes to remove undissolved substances. The resulting solution is centrifuged at 4°C, 18,000 r.p.m. for 8 hours, and the resultant precipitate is re-dissolved in 10 ml of a buffer (pH 7.5) of 10 mM Tris-HCl, 0.1 M NaCl and 1 mM EDTA. The solution is added to the top of a centrifuge tube containing 30% sucrose, which is centrifuged at 4°C, 39,000 r.p.m. for 4 hours. The resultant precipitate is redissolved in the same buffer as above.

In order to allow the following operations the buffer solution is subjected to a reaction with HBV DNA polymerase by treating it in a mixture (500 µl) of 67 mM Tris-HCl (pH 7.5), 80 mM NH₄Cl, 25 mM MgCl₂, 0.5% NP40 (Tergitol®, manufactured by Sigma Co.), 0.1%.

2-mercaptoethanol, 330 µM dCTP (deoxycytidine triphosphate), dGTP (deoxyguanosine triphosphate), and dATP (deoxyadenosine triphosphate), 0.5 µM α-[³²P]dTTP (deoxythymidine triphosphate) at 37°C for 3 hours. The same volume of a 100 mM EDTA solution is added to the reaction mixture. By the above DNA polymerase reaction, the single-stranded region of the DNA is repaired to a completely double-stranded structure which is [³²P] labeled. This DNA is added to the top of a centrifuge tube wherein 30%, 20% and 10% aqueous solutions of sucrose are packed in layers in this order, and it is centrifuged at 4°C, 39,000 r.p.m. for 4.5 hours.

In order to digest the proteins strongly bonded to DNA, the precipitates obtained above are treated in a mixture (200 µl) of 1 mg/ml of Pronase E (manufactured by Kaken Kagaku K.K.) and 0.2% aqueous sodium laurate solution at 37°C for 2 hours. The resulting mixture is extracted with phenol (200 µl) twice, and the resulting DNA-containing extract is washed with ether to remove the phenol and to give a solution of HBV DNA. The DNA thus obtained has a specific radioactiv-

ity of $2.5 \times 10^6$ cpm/μg and can be used for digestion with restriction enzymes.

(ii) Cloning of HBV DNA

The double-stranded circular HBV DNA obtained above is cloned by using λ-phage Charon 16A DNA as a vector and is then subcloned by using the known plasmid pBR322 as a vector as follows.

(A) Cloning in the system of λ-phage Chacon 16A host-vector:

HBV DNA (20 ng) is treated with endonuclease Xho I in a mixture (20 μl) of 10 mM Tris-HCl (pH 7.4), 7 mM MgCl₂, 100 mM NaCl and 7 mM 2-mercaptoethanol at 37°C for 2 hours. The resulting mixture is extracted with phenol (20 μl) and further with ether, and a double volume of cooled ethanol is added to the aqueous layer to precipitate the DNA. The mixture is kept at −70°C for one hour and then centrifuged at 10,000 r.p.m. for 5 minutes, and the precipitated DNA is recovered. The precipitate thus separated is dissolved in a mixture (5 μl) of 10 mM Tris-HCl (pH 7.4) and 1 mM EDTA. The HBV DNA and an equimolar amount of λ-phage Charon 16 A DNA (having a unite Xho I recognition site) obtained by cleavage with endonuclease Xho I in the same manner as described above are reacted with T4 DNA ligase [a mixture of 50 mM Tris-HCl (pH 7.4), 10 mM MgCl₂, 10 mM dithiothreitol, 100 μg/ml calf serum albumin, 0.5 mM ATP and 0.5 μl enzyme preparation (T4 DNA ligase, manufactured by Takara Biomedicals, 1—5×10³ unit/ml)] at 4°C for 18 hours. The reaction mixture is extracted with phenol and ether and then subjected to precipitation with ethanol in the same manner as described above. The precipitates thus obtained are dissolved in a mixture (10 μl) of 10 mM Tris-HCl (pH 7.4) and 1 mM EDTA.

The thus annealed DNA is subjected to in vitro packaging to form λ-phages in the same manner as described in "Methods in Enzymology", *68*, 299—309. Subsequently plaques (10⁴) are formed on an L-agar plate (23 cm×23 cm) by using said λ-phages and *E. coli* DP50 SupF (cf. Blattner, F. R. et al, Science (*196*, 161, 1977) as an indicator. These plaques are subjected to a plaque hybridization using ³²P-labeled HBV DNA as prepared above as a probe (cf. Science, *196*, 180, 1977) in order to select plaques formed by phases containing HBV DNA. A plurality of the desired phages are separated.

(B) Subcloning by using plasmid pACYC177 as a vector:

A phage DNA is prepared from HBV DNA cloned in Charon 16 A as obtained in step (A) by using *E. coli* DP50-SupF as bacteria to be infected in the same manner as described in "Methods in Enzymology", *68*, 245—378, 1979. The DNA thus obtained is digested with Xho I under the same conditions as described above for 2 hours, and the resulting reaction mixture is subjected to an electrophoresis with 0.75% agarose gel to isolate HBV DNA (3.2 kb). The HBV DNA is absorbed onto DEAE (diethymaminoethyl cellulose) paper (manufactured by Toyo Roshi, Japan) in order to separate it from the vector DNA and then eluted with 1 M NaCl aqueous solution. The HBV DNA thus obtained is treated with T4 DNA ligase under the same conditions as described above to give a circular HBV DNA (3.2 kb). This HBV DNA is treated with BamHI to give a BamHI fragment of HBV DNA.

Separately, *E. coli* pBR322 having a single BamHI cleavage site within its tetracycline-resistance gene is cleaved with BamHI, and the product is purifiud by phenol extraction and ethanol precipitation in the same manner as described above.

The thus obtained pBR322 cleaved with BamHI is mixed with the BamHI fragment of HBV DNA obtained above in a molar ratio of 1:5, and the mixture is annealed with T4 DNA ligase for 18 hours as described above.

The annealed DNA preparation (10 μl) obtained above is added to a solution of *E. coli* (0.1 ml) which is prepared by treating a culture broth of *E. coli* X1776 (cf. R. Curtiss III et al, "Molecular Cloning of Recombinant DNA" ed. Scott, W. A. and Werner, R., page 99, Academic Press, 1977) by the procedure as described in Norgard, M. V., Gene, *3*, 279 (1978), and the mixture is mixed well and allowed to stand at 0°C for 25 minutes. The mixture read out on an L-agar plate containing ampicillin (20 μg/ml), α-biotine (1 μg/ml), diaminopimelic acid (100 μg/ml) and thymine (20 μg/ml) and is incubated at 37°C overnight. The resulting colonies are spread out onto both an agar plate containing tetracyline (20 μg/ml) and an agar plate containing ampicillin (20 μ/ml), and the colonies which grow only on the agar plate containing ampicillin are selected. pBR322 has an ampicillin-resistance gene and a tetracyline-resistance gene, but when HBV DNA is inserted into the BamHI site of the tetracycline-resistance gene, the tetracycline resistance gene is inactivated. Accordingly, the selected colonies contain a recombinant pBR322-HBV DNA. From the colonies thus selected, a plasmid is prepared by the procedure as described by K. Matsubara (cf. J. Virol., (16, 479, 1975). The plasmid thus obtained, i.e. the recombinant pBR322-HBV DNA (linked at the BamHI site), is treated with BamHI under the same conditions as described above, and the reaction mixture is subjected to an eletrophoresis with 0.75% agarose gel in the same manner as described above to give a BamHI fragment of HBV DNA.

(2) Preparation of the vector (pXRIIG BamHI fragment)

A vector pXRIIG (1 μg) is added to a mixture (20 μl) of 10 mM Tris-HCl (pH 8.0), 7 mM MgCl₂, 100 mM NaCl and 2mM 2-mercaptoethanol, one unit of BamHI (one unit: an enzymatic activity being capable of completely cleaving 1 μg of λ-DNA per one hour) is added, and the mixture is reacted at 30°C for one hour. The reaction mixture is extracted with phenol, the aqueous layer is extracted with ether and then subjected to ethanol precipitation. The precipitates are dissolved in water and the solution is used for the preparation of a recombinant DNA.

(3) Preparation of a recombinant DNA of HBV DNA-pXRIIG

A solution (50 µl) containing the BamHI fragment (150 ng), of HBV DNA BamHI fragment (50 ng) of pXRIIG is reacted with T4 DNA ligase at 16°C for 4 hours.

*E. coli* X1776 is transformed with the obtained ligation mixture in the same manner as described above. Colonies which grow on an agar medium are selected from the resulting transformants after incubating them on L-agar plates for 12 hours in the same manner as described above in (1), (B). The colonies thus selected are spread out on an agar medium containing tetracycline (Tc) (10 µg/ml) and an agar medium containing ampicillin (Ap) (40 µg/ml). The colonies (clones) which can not grow on the Tc-containing agar medium but can grow on the Ap-containing agar medium are selected. These clones are each incubated in the above mentioned culture liquid for *E. coli* X1776, and the plasmids are extracted in the same manner as described above. By analysis of the cleavage pattern with various restriction enzymes (e.g. BamHI, Xhol, Hind III, Sal I), a recombinant DNA consisting of three HBV DNA BamHI fragments and one pXRIIG fragment is selected. It is designated as pSHB3.

(4) Transformation of mammalian cells

The following liquids A and B are prepared.

Liquid A: a solution (1.25 ml, pH 7.1) consisting of 50 mM Hepes (i.e. N-2-hydroxyethylpiperazine-N-2-ethanesulfonic acid), 280 mM NaCl, and 15 mM $Na_2HPO_4 \cdot 12H_2O$.

Liquid B: a solution containing a mixture of DNA (1.1 ml) consisting of pSHB3 (50 µg), pTK (2.5 µg) (cf. Colbere-Garapin, F., Proc. Natl. Acad. Scie. USA, *76*, 3755, 1979), salmon sperm DNA (carrier DNA) (50 µg) and 0.24 M $CaCl_2$.

The liquid B is added dropwise with stirring to the liquid A, and the mixture is allowed to stand at room temperature for 30 minutes. After pipetting sufficiently, the mixture (0.5 ml) is added dropwise to a single layer of mouse LTK⁻ cells (about $10^5$ cells/flask) in a flask. The flask is kept at room temperature for 30 minutes in order to absorb the mixture into the cells. Then DMEM (5 ml) is added and the mixture is incubated under 5% $CO_2$ at 37°C for about 5 hours. After exchanging with new DMEM, the mixture is further incubated for about 24 hours, and then, the medium is replaced by HAT medium. The incubation is continued while the medium is replaced by a new HAT medium every two to three days. After 4 weeks, the colonies of TK⁺ cells are collected to give the desired transformed cells.

(5) Production of HBV proteins

The culture liquid of the transformed mouse L cells (LTK⁺) obtained in (4) above is assayed with a kit for detecting HBsAg, HBeAg and HBcAg (manufactured by Abbott, U.S.A.). As a result, HBsAg and HBeAg are detected, but HBcAg is not detected.

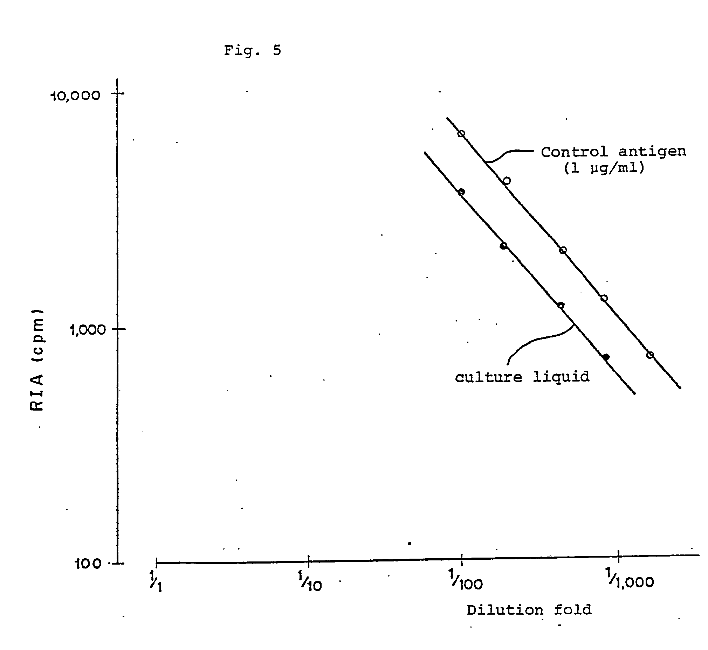

As to the culture liquid obtained above, the reactivity and amount of antigen are assumed in accordance with a parallel line assay using a kit for detecting HBsAg as above (cf. Finnery, D. J., "Statistical method in biological assay, 2nd edn. Griffin, London, 1964), wherein purified HBsAg obtained from human blood serum is used as control antigen. The results are shown in Figure 5. As is clear from Figure 5, the amount of antigen in the culture liquid of the transformed mouse cells is 600 ng/ml. Moreover, based on the parallelism with the control antigen, it is also clear that the HBsAg produced by the present invention has similar reactivities (antigenicity, immunogenicity, etc.) to the HBsAg present in human blood plasma.

Cesium chloride (1.5 g) is added to the culture liquid (5 ml), obtained above, and the mixture is centrifuged at 4°C, $200,000 \times g$ for 60 hours. Fractions having a maximum antigenicity of HBsAg and HBeAg are separated. When the specific gravity of these fractions is measured, the fraction having a maximum antigenicity of HBsAg has a specific gravity of about 1.20 and the fraction of HBeAg of about 1.28. These specific gravities are almost the same as those of HBsAg and HBeAg present in human plasma.

Besides, the transformed cells are set with carbon tetrachloride at 4°C for 30 minutes and reacted with rabbit anti-HBs anti-serum (as the primary antibody), followed by washing and drying, and then reacted with a fluorescent pigment-labeled anti-rabbit IgG antibody (as the secondary antibody), followed by washing and drying. The resulting cells are observed by fluorescent microscopy, As a result, there is observed a specific fluorescence within the cytoplasm of the transformed cells.

The culture liquid (100 µl) obtaiend above is reacted with rabbit anti-HBs antibody (antibody value PHA $2^8$) (100 µl) at room temperature for 12 hours and the reaction mixture is centrifuged at $10,000 \times g$ for 30 minutes. The resulting precipitates are stained with uranyl acetate and observed with an electron microscope. As a result, there is observed a multitude of aggregated images of particles (particle size 22 nm) similar to HBsAg particles present in human plasma which are treated likewise. Tubular particles or Dane particles (HBV) are not observed.

Thus, the transformed mouse L cells produce and release HBsAg and HBeAg into the medium.

The HBsAg and HBeAg thus produced are isolated from the culture liquid in the following manner.

The culture liquid (100 ml) is passed through a column packed with guinea pig anti-HBs antibodies linked to Sepharose CL 4B (manufactured by Pharmacia, Sweden), by which only HBsAg is adsorbed, and hence, the HBsAg can be isolated by an elution with a 0.1 M HCl-glycine buffer (pH 2.5).

The HBsAg thus obtained was subcutaneously inoculated into guinea pigs (female, 300—400 g, 10 animals) once a week for three weeks and once more after an additional month. The antibody in the blood plasma was measured with a kit for the detection of anti-HBs antibodies (AUSAB, manufactured by Abbott, U.S.A.). As a result, the same increased antibody value was observed in all animals as in a control animal which was positive for the antibody with the same kit.

Besides, the liquid passed through the above column is passed through a column packed with guinea pig anti-HBe antibodies linked to Sepharose CL 4B, by which only HBeAg is adsorbed, and the HBeAg is isolated by elution with a 0.1 M HCl-glycine buffer (pH 2.5).

## Claims

1. A recombinant DNA molecule capable of replication in mammalian cells, which comprises 3 fragments of Hepatitis B virus (HBV) DNA recombined with a vector consisting essentially of a 0.31 kb fragment of the DNA of SV40 containing the origin of replication inserted into the EcoRI cleavage site of the *Escherichia coli* plasmid pBR322 which is deficient in the 1.426—2.521 kb region inhibiting replication in mammalian cells and an antibiotic resistance gene as a selective marker useful in the method of preparing transformed mammalian cells, wherein each HBV DNA fragment is a 3.2 kb BamHI fragment consisting of the 1.9 kb HBc gene and the 1.3 kb HBs gene, and said HBV DNA fragments are arranged in the vector in a head-to-tail tandem relationship wherein the HBc gene is located at the head and the HBs gene is located at the tail.

2. The recombinant DNA according to claim 1, wherein the HBV DNA fragments recombined with the vector are BamHI fragments of the genome of the HBV subtype adr.

3. The recombinant DNA according to claim 1, which additionally comprises a DNA fragment encoding a thymidine kinase.

4. A mammalian cell being transformed with a recombinant DNA molecule according to any one of claims 1 to 3.

5. The mammalian cell according to claim 4, which is thymidine kinase deficient.

6. The mammalian cell according to claim 4, which is a mouse LTK-cell.

7. The mammalian cells according to claim 5, which is obtained by treating a mammalian cell with the recombinant DNA according to claim 1 or 2 together with a thymidine kinase DNA.

8. A method for the simultaneous production of the Hepatitis B virus proteins HBsAg and HBeAg, which comprises incubating a mammalian cell according to any one of claims 4 to 7 and collecting the produced Hepatitis B virus proteins.

## Patentansprüche

1. Rekombinantes DNA-Molekül, das zur Replikation in Säugerzellen fähig ist, enthaltend 3 Hepatitis B-Virus (HBV) DNA-Fragmente, die mit einem Vektor rekombiniert sind, der im wesentlichen besteht aus einem den Replikationsursprung enthaltenden 0,31 kb SV40 DNA-Fragment, das inseriert ist in die EcoRI-Schnittstelle des *Escherichia coli*-Plasmids pBR322, in dem die die Replikation in Säugerzellen inhibierende Region zwischen 1,426—2,521 kb defizient ist, und aus einem Antibiotikum-Resistenz-Gen als nützlichen selektiven Marker im Verfahren zur Herstellung von transformierten Säugerzellen, wobei jedes HBV DNA-Fragment ein 3,2 kb BamHI-Fragment ist, das aus dem 1,9 kb HBc und dem 1,3 kb HBs-Gen besteht, und die HBV DNA-Fragmente tandemartig in einer Kopf-zu-Schwanz-Beziehung im Vektor angeordnet sind, wobei das HBc-Gen kopfseitig und das HBs-Gen schwanzseitig liegt.

2. Rekombinante DNA nach Anspruch 1, in der die mit dem Vektor rekombierten HBV-DNA-Fragmente BamHI-Fragmente des Genoms des HBV-Subtyps adr sind.

3. Rekombinante DNA nach Anspruch 1, die ein zusätzliches DNA-Fragment aufweist, das eine Thymidin-Kinase codiert.

4. Säugerzelle, die mit einem rekombinanten DNA-Molekül nach einem der Ansprüche 1 bis 3, transformiert ist.

5. Säugerzelle nach Anspruch 4, die Thymidin-Kinase-defizient ist.

6. Säugerzelle nach Anspruch 4, die eine Maus LTK-Zelle ist.

7. Säugerzelle nach Anspruch 5, die man durch Behandlung einer Säugerzelle mit der rekombinanten DNA nach Anspruch 1 oder 2 zusammen mit einer Thymidin-Kinase-DNA erhält.

8. Verfahren zur gleichzeitigen Herstellung der Hepatitis B-Virus Proteine HBsAg und HBeAg, bei dem man eine Säugerzelle nach einem der Ansprüche 4 bis 7 inkubiert und die entstandenen Hepatitis B-Virus-Proteine gewinnt.

## Revendications

1. Molécule d'ADN recombinant capable de réplication dans des cellules de mammifère, qui comprend 3 fragments d'ADN du virus de l'hépatite B (HBV) recombinés avec un vecteur constitué essentiellement d'un fragment de 0,31 kb de l'ADN de SV40 contenant l'origine de réplication insérée dans le site de clivage Ecor I du plasmide pBR322 *d'Escherichia coli* dépourvu de la région de 1,426—2,521 kb inhibant la réplication dans les cellules de mammifère, et un gène de résistance aux antibiotiques qui est un marqueur sélectif utile dans le procédé de préparation des cellules de mammifère transformées, où chaque fragment d'ADN d'HBV est un fragment BamH I de 3,2 kb constitué du gène HBc de 1,9 kb et du gène HBs de 1,3 kb, et lesdits fragments d'ADN d'HBV sont disposés dans le vecteur en relation tandem tête-à-queu où le gène HBc est situé à la tête et le gène HBs est situé à la queue.

2. ADN recombinant selon la revendication 1, dans lequel les fragments d'ADN d'HBV recombinés avec le vecteur sont des fragments BamH I du génome du sous-type adr de l'HBV.

3. ADN recombinant selon la revendication 1, qui comprend de plus un fragment d'ADN codant pour une thymidine kinase.

4. Cellule de mammifère transformée avec une molécule d'ADN recombinant selon l'une quelconque des revendications 1 à 3.

5. Cellule de mammifère selon la revendication 4, qui est déficiente en thymidine kinase.

6. Cellule de mammifère selon la revendication 4, qui est une cellule LTK de souris.

7. Cellule de mammifère selon la revendication 5, qui est obtenue par traitement d'une cellule de mammifère avec l'ADN recombinant selon la revendication 1 ou 2 avec un ADN codant pour la thymidine kinase.

8. Procédé pour la production simultanée des protéines HBsAg et HBeAg du virus de l'Hépatite B, qui comprend l'incubation d'une cellule de mammifère selon l'une quelconque des revendications 4 à 7 et la récolte des protéines du virus de l'hépatite B produites.

Fig. 1

Fig. 2

1

Fig. 3A

Fig. 3B

Fig. 4

Fig. 5